# EUROPÄISCHE PATENTSCHRIFT

(11) **EP 3 049 156 B1**
(45) Veröffentlichungstag und Bekanntmachung des Hinweises auf die Patenterteilung: **10.01.2018**
(21) Anmeldenummer: 14771313.5
(22) Anmeldetag: 19.09.2014
(51) Int. Cl.: A61Q 3/02, A61K 8/85, A61K 8/86, A61K 8/37, A61K 8/49, A61K 8/73

(54) **NEUE WEICHMACHER FÜR NAGELLACKE**
NEW SOFTENING AGENTS FOR NAIL VARNISHES
NOUVEAUX PLASTIFIANTS POUR VERNIS À ONGLES

(30) Priorität: 23.09.2013 EP 13185609
(43) Veröffentlichungstag der Anmeldung: 03.08.2016
(73) Patentinhaber: Covestro Deutschland AG, 51373 Leverkusen (DE)
(72) Erfinder: VIALA, Sophie, 50935 Köln (DE); DÖRR, Sebastian, 40593 Düsseldorf (DE)
(74) Vertreter: Levpat
(86) Internationale Anmeldenummer: PCT/EP2014/070015
(87) Internationale Veröffentlichungsnummer: WO 2015/022438

(56) Entgegenhaltungen:
- WO-A1-00/54732
- KR-B1- 101 044 853
- US-A- 3 117 102
- US-A- 4 626 428
- US-A- 5 807 540
- US-A1- 2007 243 149

## Beschreibung

Die vorliegende Erfindung betrifft eine Nagellackzusammensetzung, enthaltend einen Carbonatstruktureinheiten enthaltenden Weichmacher A) und einen Filmbildner B). Weitere Gegenstände der Erfindung sind eine Beschichtung erhältlich aus der erfindungsgemäßen Nagellackzusammensetzung, ein Substrat beschichtet mit einer solchen erfindungsgemäßen Beschichtung, sowie ein kosmetisches Verfahren zum Beschichten von Finger- und/oder Fußnägeln, bei dem eine erfindungsgemäße Nagellackzusammensetzung auf Finger- und/oder Fußnägel aufgetragen wird. Weiterhin ist die Verwendung von speziellen Carbonatstruktureinheiten enthaltenden Verbindungen als Weichmacher in Nagellackzusammensetzungen Gegenstand dieser Erfindung.

Kosmetische Formulierungen müssen einige Eigenschaften aufweisen, um als Nagellacke geeignet zu sein. Dabei ist es besonders wichtig, dass Haut und Nägel nicht gereizt werden, die Formulierung leicht angewendet werden kann, sich beim Auftragen homogene und glänzende Filme bilden und diese schnell trocknen. Zudem muss der Film gut auf der Nageloberfläche haften, möglichst flexibel sein, sowie eine gute Schlagzähigkeit und eine gute Verschleißfestigkeit aufweisen, um eine Rissbildung und ein Abblättern des Nagellacks zu verhindern. Dazu ist es notwendig, dass die kosmetische Formulierung in der Lage ist einen harten, aber auch flexiblen Film zu bilden. Vorteilhaft ist auch eine gute Beständigkeit des ausgehärteten Films gegenüber Wasser, um ein Ablösen vom Nagel bei Kontakt mit Wasser, beispielsweise beim Händewaschen oder Spülen, zu vermeiden.

Weichmacher verbessern dabei in Nagellackformulierungen die Flexibilität der Filme und beeinflussen auch andere wichtige Parameter wie den Glanz, die Trocknungszeit und die Haftung auf den Nägeln. Lange wurden fast ausschließlich Phthalate, wie Dibutylphthalat oder Kampfer, oder polymere Kondensationsprodukte von Formaldehyd oder anderen Aldehyden als Weichmacher in Nagellackformulierungen eingesetzt. Da sich diese Substanzen jedoch als gesundheitsschädlich erwiesen haben, besteht ein Bestreben diese in Nagellackformulierungen zu ersetzen.

Die US 5,227,155 erwähnt erstmals den Einsatz von Acetyltributylcitrat (ATC) als Weichmacher in Nagellackformulierungen. Diese Substanz hat heute bereits weitestgehend die oben genannten gesundheitsgefährdenden Weichmacher ersetzt. Allerdings führt ATC zu einem deutlich verringerten Glanz im Vergleich zu den zuvor verwendeten Weichmachern. Darüber hinaus neigt ATC auf Grund seiner niedrigen Molmasse dazu aus dem Nagellackfilm heraus zu migrieren und sich an der Oberfläche des Films abzulagern. Dadurch sieht die Oberfläche des Films Stumpf aus und der Weichmachereffekt im Nagellack selbst geht verloren. Zudem härten Nagellackformulierungen mit ATC als Weichmacher nur langsam aus.

Oberfläche des Films Stumpf aus und der Weichmachereffekt im Nagellack selbst geht verloren. Zudem härten Nagellackformulierungen mit ATC als Weichmacher nur langsam aus.

In der WO 03/094870 A wird unter anderem Propylencarbonat als potentieller Weichmacher für Nagellackformulierungen erwähnt. Allerdings zeigt Propylencarbonat in Nagellackformulierungen einen deutlich zu geringeren Weichmachereffekt um sinnvoll und effizient eingesetzt werden zu können. Zudem ist Propylencarbonat flüchtig und kann aus dem Nagellackfilm heraus migrieren.

Die US 2010/0158835 A1 beschreibt bestimmte niedermolekulare Carbonate, wie beispielsweise Glycerincarbonat, als Weichmacher für Nagellackformulierungen. Bei diesen Weichmachern ist jedoch bekannt, dass der ausgehärtete Nagellack eine schlechte Beständigkeit gegenüber Wasser aufweist. Dies zeigt sich durch ein Aufquellen oder Zersetzen des Nagellacks und einer verringerten Haftung des Nagellacks auf dem Nagel. Zudem hörten Nagellackformulierungen mit Glycerincarbonat als Weichmacher etwas zu langsam aus.

Es bestand daher ein Bedarf nach Nagellackzusammensetzungen, die Weichmacher enthalten, die keine toxikologisch bedenklichen Eigenschaften zeigen, nicht flüchtig sind und zudem nur eine geringe Migrationsfähigkeit aus dem Film heraus oder durch Gewebe und Haut aufweisen. Zudem sollten die Nagellackzusammensetzungen schnell aushärten und eine gute Wasserbeständigkeit aufweisen.

Aufgabe der vorliegenden Erfindung war es daher, eine Nagellackzusammensetzung bereitzustellen, die schnell aushärtet, eine gute Wasserbeständigkeit aufweist und auf Weichmachern basiert, die toxikologisch unbedenklich und nicht flüchtig sind, sowie eine geringe Migrationsfähigkeit aufweisen.Diese Aufgabe wurde gelöst durch eine Nagellackzusammensetzung, enthaltend einen Weichmacher A) und einen Filmbildner B), dadurch gekennzeichnet, dass der Weichmacher A) mindestens zwei Carbonatstruktureinheiten der Formel (I), ein zahlenmittleres Molekulargewicht von ≥ 450 g/mol und ≤ 6000 g/mol, bestimmt durch Gelpermeationschromatographie (GPC) gegenüber Polystyrol-Standards in Tetrahydrofuran bei 23°C gemäß DIN 55672-1, und eine Hydroxylfunktionalität von ≥ 1,5 und ≤ 6 aufweist, wobei der Weichmacher A) keine chemische Reaktion mit anderen Komponenten der Nagellackzusammensetzung eingeht

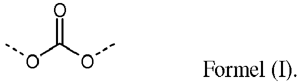

Das zahlenmittlere Molekulargewicht wird im Rahmen dieser Anmeldung stets bestimmt durch Gelpermeationschromatographie (GPC) gegenüber Polystyrol-Standard in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2xPSS SDV linear M, 8x300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Überraschend wurde nun gefunden, dass sich polymere Verbindungen enthaltend mindestens zwei Carbonatstruktureinheiten hervorragend als Weichmacher für Nagellackzusammensetzungen eignen. Diese Verbindungen bieten den Vorteil, dass sie nicht flüchtig und toxikologisch unbedenklich sind. Zudem weisen sie auf Grund ihrer polymeren Struktur nur eine geringe Migrationsfähigkeit aus dem Nagellackfilm heraus oder durch Gewebe und Haut auf. Die erfindungsgemäßen Nagellackzusammensetzungen härten zudem schnell aus und weisen eine besonders gute Wasserbeständigkeit auf.

Unter Weichmachern werden im erfindungsgemäßen Sinne Stoffe verstanden, die die aus einer Nagellackformulierung gebildeten Filme weicher, flexibler, geschmeidiger und elastischer machen. Es handelt sich ausschließlich um eine *sogenannte äußerte Weichmachung,* was bedeutet, dass die Weichmachermoleküle nicht kovalent an andere in der Nagellackformulierung vorhanden Moleküle, insbesondere Polymere, angebunden sind oder während der Filmbildung oder Aushärtung angebunden werden.

Ein weiterer Gegenstand der Erfindung ist die Verwendung von Verbindungen, die mindestens zwei Carbonatstruktureinheiten der Formel (I), ein zahlenmittleres Molekulargewicht von ≥ 450 g/mol und ≤ 6000 g/mol, bestimmt durch Gelpermeationschromatographie (GPC) gegenüber Polystyrol-Standard in Tetrahydrofuran bei 23°C gemäß DIN 55672-1, und eine Hydroxylfunktionalität von ≥ 1,5 und ≤ 6 aufweisen, als Weichmacher in Nagellackzusammensetzungen, wobei der Weichmacher A) keine chemische Reaktion mit anderen Komponenten der Nagellackzusammensetzung eingeht.

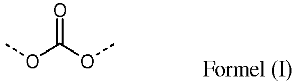

Das zahlenmittlere Molekulargewicht wird dabei wie schon vorstehend beschrieben wurde bestimmt.

Für die in diesem Kontext genannten Nagellackzusammensetzungen und als Weichmacher geeigneten Verbindungen gelten analog die zu der erfindungsgemäßen Nagellackformulierung und den dort eingesetzten Weichmachern A) angeführten Ausführungsformen.

Erfindungsgemäß geht der Weichmacher A) keine chemische Reaktion mit anderen Komponenten der Nagellackzusammensetzung ein. Der Weichmacher A) liegt also bevorzugt in der Zusammensetzung und im ausgebildeten und/oder ausgehärteten Nagellackfilm chemisch unverändert vor. Der Weichmacher A) ist oder wird daher nicht kovalent an andere in der Nagellackzusammensetzung vorhandene Moleküle, insbesondere Polymere angebunden.

Die erfindungsgemäße Zusammensetzung enthält einen Weichmacher A), der mindestens zwei, bevorzugt mindestens drei und besonders bevorzugt mindestens vier Carbonatstruktureinheiten der Formel (I) aufweist.

Der erfindungsgemäß eingesetzte Weichmacher A) weist ein zahlenmittleres Molekulargewicht von ≥ 450 g/mol und ≤ 6000 g/mol, bevorzugt von ≥ 500 g/mol und ≤ 5000 g/mol und besonders bevorzugt von ≥ 600 g/mol und ≤ 3000 g/mol auf.

Der Weichmacher A) enthält weiterhin Hydroxylgruppen. Dabei weist er eine Hydroxylfunktionalität von ≥ 1,5 und ≤ 6, bevorzugt von ≥ 1,8 und ≤ 3 und besonders bevorzugt von ≥ 1,9 und ≤ 2,1 auf.

Unter Hydroxylfunktionalität ist dabei die mittlere Anzahl der Hydroxylgruppen pro Molekül, also die Anzahl der endständigen Hydroxylgruppen/Anzahl der Moleküle zu verstehen.

Die Hydroxylgruppen liegen in der Zusammensetzung bevorzugt frei vor und gehen keine chemische Reaktion mit anderen Komponenten der Zusammensetzung ein.

Bevorzugt weist der Weichmacher A) keine Urethan und/oder Harnstoffgruppen auf. Ebefalls bevorzugt weist der Weichmacher A) keine aromatischen Struktureinheiten auf.

Bevorzugt ist der Weichmacher A) ausgewählt aus Polycarbonatpolyolen, Polyestercarbonatpolyolen und/oder Polyethercarbonatpolyolen oder Gemischen daraus, besonders bevorzugt aus Polycarbonatpolyolen und/oder Polyestercarbonatpolyolen und ganz besonders bevorzugt ist der Weichmacher A) ein Polyestercarbonatpolyol. In einer bevorzugten Ausführungsform der Erfindung handelt es sich um aliphatische Polycarbonatpolyolen, Polyestercarbonatpolyolen und/oder Polyethercarbonatpolyolen, die keine aromatischen Struktureinheiten aufweisen, besonders bevorzugt um aliphatische Polycarbonatpolyolen und/oder Polyestercarbonatpolyolen und ganz besonders bevorzugt um aliphatische Polyestercarbonatpolyole.

Ganz besonders bevorzugt handelt es sich bei den Weichmachern A) um Polycarbonatpolyole und/oder Polyestercarbonatpolyole mit einer Hydroxylfunktionalität von ≥ 1,9 und ≤ 2,1 und einem zahlenmittleren Molekulargewicht von ≥ 600 g/mol und ≤ 3000 g/mol.

Erfindungsgemäß einsetzbare Polycarbonatpolyole sind beispielsweise erhältlich durch Reaktion von Kohlensäurederivaten, wie Diphenylcarbonat, Dimethylcarbonat oder Phosgen, mit Polyolen, vorzugsweise Diolen. Als derartige Diole kommen beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Bisphenol A und Tetrabrombisphenol A, sowie deren Gemische in Frage.
Bevorzugt enthält die Diolkomponente 40 bis 100 Gew.-% Hexandiol, bevorzugt 1,6-Hexandiol und/oder Hexandiol-Derivate. Bevorzugt sind solche Polycarbonatpolyole auf Basis von 1,6-Hexandiol, besonders bevorzugt solche auf Basis von Mischungen aus 1,6-Hexandiol und 1,4-Butandiol.

Beispiele geeigneter Polycarbonatpolyole sind die unter den Handelsnamen Desmophen® C 2100, Desmophen® C 2200, Desmophen® C XP 2613, Desmophen® C 3100 XP, Desmophen® C 3200 XP und Desmophen® C XP 2716 erhältlichen Produkte der Bayer Material Science AG.

Erfindungsgemäß geeignete Polycarbonatpolyole sind auch solche, die neben Carbonatstrukturen zusätzlich Estergruppen enthalten und daher auch als Polyestercarbonatpolyole bezeichnet werden. Hierbei handelt es sich insbesondere um die an sich bekannten Polyestercarbonatpolyole, wie sie beispielsweise gemäß der Lehre der DE 1 770 245 A durch Umsetzung zwei- oder mehrwertiger Alkohole mit Lactonen, wie insbesondere ε-Caprolacton, und anschließende Reaktion der dabei entstehenden Polyesterdiole mit Kohlensäurederivaten, wie Diphenyl-, Dimethylcarbonat oder Phosgen erhalten werden können. Bevorzugt wird zur Herstellung der Polyesterdiole 1,6-Hexandiol mit Caprolactonen, insbesondere ε-Caprolacton umgesetzt.

Beispiele solcher geeigneter Polyestercarbonatpolyole sind die unter den Handelsnamen Desmophen® C 1100 oder Desmophen® C 1200 erhältlichen Produkte der Bayer Material Science AG.

Ebenfalls geeignete sind Polyethercarbonatpolyole, die neben Carbonatstrukturen zusätzlich Ethergruppen enthalten. Hierbei handelt es sich insbesondere um die an sich bekannten Polyethercarbonatpolyole, wie sie beispielsweise nach dem Verfahren der EP 2 046 861 A durch katalytische Umsetzung von Alkylenoxiden (Epoxiden) und Kohlendioxid in Anwesenheit von H-funktionellen Startersubstanzen erhältlich sind. Polyethercarbonatpolyole können ebenfalls durch Reaktion von Kohlensäurederivaten, wie Diphenylcarbonat, Dimethylcarbonat oder Phosgen, mit Ethergruppen haltigen Polyolen, vorzugsweise Diolen erhalten werden. Als derartige Diole kommen beispielsweise Di-, Tri- oder Tetraethylenglykol, Dipropylenglykol, Polypropylenglykole, Dibutylenglykol, Polybutylenglykole und Polytetramethylenetherglykole, sowie deren Gemische in Frage. Des Weiteren bevorzugt werden, wie aus der DE 2 650 533 A bekannt ist, zur Herstellung der Polyethercarbonatpolyole Polyalkylenoxidpolyole, insbesondere Polyalkylenoxiddiole als Alkohole eingesetzt. Weiterhin bevorzugt eingesetzt werden auch solche Diole, die durch Veretherung von 1,6-Hexandiol mit anderen Diolen, insbesondere mit sich selbst zum Di- oder Trihexylenglykol, erhalten wurden. Die Herstellung derartiger Polyethercarbonatpolyole ist beispielsweise aus der EP 0 292 772 A bekannt.

Die Polycarbonatpolyole, Polyestercarbonatpolyole und/oder Polyethercarbonatpolyole sind bevorzugt im Wesentlichen linear. Sie können jedoch gegebenenfalls durch den Einbau polyfunktioneller Komponenten mit einer OH-Funktionalität > 2, insbesondere niedermolekularer Polyole, leicht verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Im Wesentlichen linear heißt dabei, dass bevorzugt 0 bis 10 Mol% der Alkoholbausteine mit einer OH-Funktionalität > 2 zur Herstellung von A), besonders bevorzugt 0 bis 3Mol% und ganz besonders bevorzugt überhaupt keine Bausteine mit einer OH-Funktionalität > 2 eingesetzt werden.

Der Anteil des Weichmacher A) beträgt bevorzugt ≥ 0,1 und ≤ 20 Gew.-%, besonders bevorzugt ≥ 0,5 und ≤ 15 Gew.-% und ganz besonders bevorzugt ≥ 1 und ≤ 10 Gew.-% an der gesamten Nagellackformulierung.

In einer bevorzugten Ausführungsform der Erfindung enthält die Nagellackzusammensetzung keine Verbindungen die Isocyanatgruppen enthalten.

Die erfindungsgemäße Zusammensetzung enthält als weitere Komponente einen Filmbildner B), wobei es sich bevorzugt um ein filmbildendes Polymere handelt. Dieser Filmbildner B) kann auch als primärer Filmbildner B) bezeichnet werden. Bevorzugt handelt es sich um einen kosmetischen Filmbildner.
Das primäre filmbildende Polymer ist vorteilhaft ausgewählt aus Nitrocellulosen, Celluloseacetobutyraten, Celluloseacetopropionaten, Celluloseethern und/oder Gemischen davon. Besonders bevorzugt sind Nitrocellulosen. Ganz besonders bevorzugt sind die unter dem Grad RS kommerziell verfügbaren Nitrocellulosen mit einem Stickstoffgehalt zwischen 11,2 und 12,8%. Innerhalb des RS-Grades, gibt es Sub-Typen, die sich insbesondere durch ihr Molekulargewicht unterscheiden. Weiterhin bevorzugt werden Nitrocellulose RS ⅛ sec., Nitrocellulose ¼ sec., Nitrocellulose RS½ sec., Nitrocellulose RS 5-6 sec. und Nitrocellulose 60-80 sec. in der erfindungsgemäßen Zusammensetzung verwendet. Diese Nitrocellulose sind unter der Europäischen Nomenklatur als Nitrocellulose E 23 bis Nitrocellulose E 130 bekannt.

Der Anteil des Filmbildner B) in der erfindungsgemäßen Zusammensetzung liegt bevorzugt bei ≥ 5 und ≤ 30 Gew.-%, besonders bevorzugt bei ≥ 6 und ≤ 25 Gew.-% und ganz besonders bevorzugt ≥ 8 und ≤ 20 Gew.-%, bezogen auf das Gesamtgewicht der Zusammensetzung.

In einer bevorzugten Ausführungsform der Erfindung enthält die Zusammensetzung mindestens einen weiteren von B) verschiedene Filmbildner D), wobei es sich besonders bevorzugt um ein filmbildendes Polymer handelt. Dieser Filmbildner D) kann auch als sekundärer Filmbildner D) bezeichnet werden.
Bevorzugt werden als sekundäre Filmbildner D) Estergruppen-haltige Polymere eingesetzt, besonders bevorzugt Polyesterpolyole.
Die bevorzugt verwendeten Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester, wobei 1,6-Hexandiol und Isomere, 1,4-Butandiol, Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Es können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Polyesterpolyole können mit einem zahlenmittleren Molekulargewicht von vorzugsweise ≥ 450 und ≤ 6000 g/mol, besonders bevorzugt > 500 und ≤ 5000 g/mol und insbesondere bevorzugt ≥ 600 und ≤ 3000 g/mol eingesetzt werden. Die Bestimmung des zahlenmittleren Molekulargewichts erfolgt mittels GPC in Tetrahydrofuran bei 23 °C gemäß den oben gemachten Ausführungen.

Bei den Polyesterpolyolen handelt es sich vorteilhaft um die von der Bayer Material Science AG unter den folgenden Handelsnamen erhältlichen Produkte oder Gemische daraus:
Desmophen® 1700, Desmophen® 1652, Desmophen® VPLS 2328, Desmophen® 850, Desmophen® 1150, Desmophen® 1155, Desmophen® 1145, Desmophen® 1800, Desmophen® 670, Desmophen® 1200, Desmophen® VPLS 2068, Desmophen® 1100, Desmophen® 2400 S, Desmophen® 2450 X, Desmophen® 800, Desmophen® VPLS 2249/1, Desmophen® 690 MPA, Desmophen® T 1665 SN/IB, Desmophen® 680 X, Desmophen® T1775 SN, Desmophen® 680 BA, Desmophen® T 2082, Desmophen® T XP 2374, Desmophen® 670 BA, Desmophen® VP LS 2388, Desmophen® 650 MPA, Desmophen®651 MPA, Desmophen® 651 MPA/X, Desmophen® 800 BA, Desmophen® 800 MPA, Desmophen® PL 800, Desmophen® PL 300 X, Desmophen® 1300 BA, Desmophen® 1300 EA, Desmophen® 1300 X, Desmophen® 1400 PR, Desmophen® PL 817, Desmophen® 1388 71 EA, Baycoll® AD 2055, Baycoll® AD 2047, Baycoll® AD 1225, Baycoll® AD 5027, Baycoll® AS 2060, Baycoll® AV 2113
Besonders vorteilhaft wird Desmophen® 680 BA eingesetzt.

Weitere vorteilhafte sekundäre Filmbildner, die zusätzlich zu oder statt den oben genannten Polyesterpolyolen eingesetzt werden können, sind:
- Tosylamid / Formaldehyd Harze (wie zum Beispiel Ketjenflex® MH und Ketjenflex® MS-80 der Firma Akzo Nobel, Sulfonex® der Firma Estron , Santolite MHP, Santolite MS 80 der Firma FACONNIER oder RESIMPOL 80 der Firma PAN AMERICANA)
- Tosylamid/ Epoxy Harze (wie zum Beispiel Lustrabrite S, Lustrabrite S-70, Nagellite 3050 der Firma Telechemische oder Polytex® Resin, Polytex® NX-55 der Firma Estron)
- Adipinsäure/Neopentylglykol/ Phthalsäureanhydrid Copolymere (wie zum Beispiel Uniplex 670-P der Firma Unitex Chempol Corporation)
- Phthalsäureanhydrid/Glycerin/Glycidyl Decanoate Copolymere
- Phthalsäureanhydrid/Phthalsäureanhydrid/Glycol Copolymere (wie zum Beispiel Polynex Resin, 7809 Polynex Resin der Firma Estron)
- Glycerin/Phthalsäure Copolymere
- Styrol/Acrylat/Acrylonitril Copolymere
- Polymere und Copolymere von Acrylaten (wie zum Beispiel ACRYLOID B66 der Firma Dow)
- Copolymeren von Acrylaten und Styrol
- Saccharoseacetatisobutyrat (wie zum Beispiel Eastman SAIB von der Firma Eastman Chemical company)
- Polyvinylbutyralharze
- Alkydharze (wie zum Beispiel BECKOSOL ODE 230-70-E der Firma DAINIPPON)
- Polyurethanharze (wie zum Beispiel TRIXENE PR 4127 der Firma Baxenden Chemicals Ltd.)

Der Mengenanteil des sekundären Filmbildners D) in der erfindungsgemäßen Zusammensetzung liegt bevorzugt bei ≥ 0 und ≤ 30 Gew.-%, besonders bevorzugt ≥ 0,5 und ≤ 20 Gew.-% und ganz besonders bevorzugt > 1 und < 15 Gew.-%, bezogen auf das Gesamtgewicht der Zusammensetzung.

Weiterhin bevorzugt liegt der Anteil der Summe der Filmbildner B) und D) bevorzugt bei bei ≥ 5 und ≤ 60 Gew.-%, besonders bevorzugt bei ≥ 10,5 und ≤ 45 Gew.-% und ganz besonders bevorzugt ≥ 11 und ≤ 35 Gew.-%, bezogen auf die gesamten Nagellackformulierung.

In einer bevorzugten Ausführungsform der Erfindung enthält die Zusammensetzung wenigstens einen weiteren, von A) verschiedenen, Weichmacher C).
Als Weichmachern C) werden vorteilhaft Weichmacher und/oder weichmachende Harze mit einem zahlenmittleren Molekulargewicht von weniger als 1500 g/mol eingesetzt, um die gewünschten mechanischen Eigenschaften zu erzielen. Als Weichmacher C) sind beispielweise Glykole und deren Ester und Ether, Estern von Säuren, insbesondere Carbonsäuren, wie Citrate, Adipate, Carbonate, Tartrate, Phosphate oder Sebacate, ethoxylierte Derivate wie ethoxylierte Öle und/oder deren Mischungen geeignet. Zum Beispiel sind geeignete Weichmacher Diethylenglykolethylether, Diethylenglykolmethylether, Diethylenglykolnbutylether oder Diethylenglykolhexylether, Ethylenglykolethylether, Ethylenglykolbutylether, Ethylenglykolhexylether, Propylenglykolphenylether, Propylenglykoldiacetat, Dipropylenglykol Ethylether, Tripropylenglykol-methylether, Diethylenglykol-methylether, Propylenglykol Butyldiglykollösung, Tributylphosphat, Tributoxyethylphosphat, Trikresylphosphat, Triphenylphosphat, Glycerintriacetat, Butylstearat, Glykolsäurebutylester, Benzylbenzoat, Butyl acetyltricinoleate, Glyceryl acetyltricinoleate, Dibutylphthalat, Diisobutylphthalat, Dioctylphthalat, Dimethoxyethylphthalat, Diethylphthalat, Diamylphthalat, Triethylcitrat, Tributylcitrat, Tributylacetylcitrate, Triethylacetylcitrate, Tri (2-ethylhexyl) Acetyl-, Dibutyltartrat, Triacetin, Kampfer, Trimethylpentanyldiisobutyrat, Triethylhexanoïme, Saccharosebenzoat, Dibutyladipat, Diisobutyladipat, Adipinsäurediisopropylester, Dipropylenglykoldibenzoat und/oder Gemischen davon.

Bestimmte Harze mit geringem Molekulargewicht können ebenfalls als Weichmacher C) eingesetzt werden und werden als externe Weichmacher betrachtet, da sie die Systeme plastifizieren, ohne die cellulosehaltigen Filmbildnerharze zu lösen. Zu nennen sind als vorteilhafte Weichmacher C) Adipat-Polyester, Sebacat-Polyester oder Butylacrylatharze.

Der Mengenanteil an weiteren Weichmachern C) in der erfindungsgemäßen Zusammensetzung liegt bevorzugt im Bereich von ≥ 0 und ≤ 25 Gew.-%, besonders bevorzugt ≥ 0,5 und ≤ 15 Gew.-% und ganz besonders bevorzugt ≥ 1 und ≤ 5 Gew.-%, bezogen auf das Gesamtgewicht der Zusammensetzung.

Weiterhin bevorzugt liegt der Anteil der Summe der Weichmacher A) und C) bevorzugt bei ≥ 0,1 und ≤ 30 Gew.-%, besonders bevorzugt > 2 und ≤ 20 Gew.-% und ganz besonders bevorzugt ≥ 3 und ≤ 12 Gew.-%, bezogen auf die gesamten Nagellackformulierung.

In einer bevorzugten Ausführungsform der Erfindung werden sowohl Weichmacher A), als auch Weichmacher C) eingesetzt. Das Gewichtsverhältnis der Weichmacher A) zu den Weichmachern C) liegt dabei bevorzugt zwischen 10:1 und 1:5, besonders bevorzugt zwischen 3:1 und 1:1.

In einer weiteren bevorzugten Ausführungsform ist in der Zusammensetzung neben A) kein weiterer Weichmacher enthalten.

Die erfindungsgemäße Nagellackzusammensetzung enthält bevorzugt des Weiteren ein physiologisch akzeptables Medium E). Daher liegen die Komponenten A) und B) und gegebenenfalls C) und D) in der erfindungsgemäßen Zusammensetzung bevorzugt in dem physiologisch akzeptablen Medium E) vor, besonders bevorzugt liegen diese Komponenten ausschließlich in dem physiologisch akzeptablen Medium E) vor.

Unter physiologisch akzeptablen Medien sind dabei Stoffe zu verstehen, die toxikologisch unbedenklich und zum Auftragen auf den menschlichen Körper, speziell auf Keratin-basierte Körperbestandteile wie Nägel, geeignet sind.

Bevorzugt enthält das physiologisch akzeptable Medium E) der erfindungsgemäßen Zusammensetzung ein organisches Lösungsmittel oder eine Mischung von organischen Lösungsmitteln, besonders bevorzugt besteht es daraus. Die bevorzugten organischen Lösungsmittel sind beispielsweise:
- Ketone, die bei Raumtemperatur flüssig sind, wie zum Beispiel Methylethylketon, Methylisobutylketon, Diisobutylketon, Isophoron, Cyclohexanon, Aceton;
- Alkohole, die bei Raumtemperatur flüssig sind, wie zum Beispiel Ethanol, Butanol, Propanol, Isopropanol, Diacetonalkohol, 2-Butoxyethanol, Cyclohexanol;
- Propylenglykole, die bei Raumtemperatur flüssig sind, wie zum Beispiel Propylenglykolmonomethylether, Propylenglykolmonomethyletheracetat, Dipropylenglykolmono-n-butylether;
- Cyclische Ether, wie zum Beispiel γ-Butyrolacton;
- Kurzkettige Ester mit 3 bis 8 Kohlenwasserstoffatomen, wie zum Beispiel Ethylacetat, Methylacetat, Propylacetat, Isopropylacetat, n-Butylacetat, Isopentylacetat, Methoxypropylacetat, t-Butylacetat, Butyllacetat;
- Ether, die bei Raumtemperatur flüssig sind, wie zum Beispiel Diethylether, Dimethylether, Dichlorodiethylether;
- Alkane, die bei Raumtemperatur flüssig sind, wie zum Beispiel Decan, Heptan, Dodecan, Hexan, Cyclohexan;
- Glykole, wie zum Beispiel Ethylenglykol, Propylenglykol, Pentylenglykol, Glycerol;
- Alkylsulfoxide, wie zum Beispiel Dimethylsulfoxide;
- Aldehyde, die bei Raumtemperatur flüssig sind, wie zum Beispiel Benzaldehyd, Acetaldehyd;
- 3-Ethylethoxypropionat;
- Carbonate, wie zum Beispiel Propylencarbonat, Dimethylcarbonat;
- Acetale, wie zum Beispiel Methylal;
und Gemische der genannten Lösungsmittel.

Besonders vorteilhaft werden die organischen Lösungsmittel ausgewählt aus der Gruppe von Ethylacetat, Butylacetat, Propylacetat, Isobutylacetat, Ethanol, Propanol, Isopropanol, Butanol, Methylethylketon, Methylisobutylketon, Heptan und/oder Hexan und Gemischen daraus.

Im Fall der erfindungsgemäße Zusammensetzung kann der Lösungsmittelanteil im Bereich von ≥ 10 und ≤ 95 Gew.-%, bevorzugt im Bereich von ≥ 15 und ≤ 80 Gew.-%, und besonders bevorzugt im Bereich von ≥ 20 und ≤ 70 Gew.-%, bezogen auf das Gesamtgewicht der Zusammensetzung, liegen.

In einer weiteren Ausführungsform der Erfindung enthält das physiologisch akzeptable Medium E) der erfindungsgemäßen Zusammensetzung Wasser und gegebenenfalls ein physiologisch verträgliches, mit Wasser mischbares organisches Lösungsmittel, wie zum Beispiel aliphatische C1-C5 Monoalkohole und C2-C8 Glykole. Das verwendete Wasser in der erfindungsgemäßen Zusammensetzung kann ein Blütenwasser, reines demineralisiertes Wasser, Mineralwasser, Thermalwasser und/oder Meerwasser sein.

Der Mengenanteil der Summe des Wassers und des gegebenenfalls vorhandenen physiologisch verträglichen, mit Wasser mischbaren organischen Lösungsmittels in der erfindungsgemäßen Zusammensetzung kann beispielsweise im Bereich von ≥ 10 und ≤ 90 Gew.-%, bevorzugt im Bereich von ≥ 15 und ≤ 80 Gew.-%, und besonders bevorzugt im Bereich von ≥ 20 und ≤ 70 Gew.-%, bezogen auf das Gesamtgewicht der Zusammensetzung, liegen.

Besonders bevorzugt enthält die erfindungsgemäße Zusammensetzung jedoch ≤ 5 Gew.-% Wasser, bezogen auf das Gesamtgewicht der Zusammensetzung.

Ferner kann die erfindungsgemäße Zusammensetzung auch Additive wie effektgebende Bestandteile, beispielsweise Farbstoffe oder Pigmente, Antioxidantien, Lichtschutzmittel, Benetzungsmittel, Emulgatoren, Dispergiermittel, Stabilisatoren, Entschäumer, Füllstoffe, Verlaufsmittel, Koaleszenzmittel, Konservierungsmittel, feuchtigkeitsspendende Substanzen, Parfum, Radikalfänger, Verdickungsmittel, Neutralisationsmittel, Öle, Wachse, Füllstoffe und Wirkstoffe enthalten. Je nach gewünschtem Eigenschaftsbild und Verwendungszweck der erfindungsgemäßen Zusammensetzung können bis zu 90 Gew.-%, bezogen auf die Gesamttrockensubstanz, dieser Additive (bezogen auf die Summe aller Additive) im Endprodukt enthalten sein. Unter der Gesamttrockensubstanz sind dabei alle nichtflüchtigen Anteile der Nagellackzusammensetzung zu verstehen.

Die erfindungsgemäße Zusammensetzung kann einen effektgebenden Bestandteil enthalten. Der genannte Bestandteil kann insbesondere farbgebend sein, aber auch sonstige andere Effekte bereitstellen, wie Glitzer- und/oder Metalliceffekte. Bevorzugt enthält die erfindungsgemäße Zusammensetzung mindestens einen Farbstoff, der bevorzugt aus der Gruppe von lipophilen Farbstoffen, hydrophilen Farbstoffen, Pigmenten, Pailletten und Perlmutt ausgewählt wird. Erfindungsgemäß besonders vorteilhaft liegt der Anteil der effektgebenden Bestandteile bei ≥ 0 und ≤ 50 Gew.-%, besonders vorteilhaft ≥ 0,5 und ≤ 30 Gew.-%, ganz besonders vorteilhaft von ≥ 1 und ≤ 15 Gew.-%, jeweils bezogen auf das Gesamtgewicht der Zusammensetzung.

Beispielweise können lipophile Farbstoffe verwendet werden, wie Sudan I (gelb), Sudan II (orange), Sudan III (rot), Sudan IV (Scharlachrot), DC Red 17, DC Green 6, β-Carotin, Sojaöl, DC Yellow 11, DC Violett 2, DC Orange 5 und DC Yellow 10.
Als Pigmente kommen prinzipiell alle anorganischen oder organischen Pigmente in Frage, die in kosmetischen oder dermatologischen Zusammensetzung verwendet werden. Die erfindungsgemäßen verwendeten Pigmente können beispielsweise weiß oder farbig sein, sie können umhüllt bzw. beschichtet sein mit einem hydrophoben Behandlungsmittel oder nicht beschichtet sein.

Vorteilhaft werden die Pigmente gewählt aus der Gruppe der Metalloxide, wie beispielsweise Eisenoxide (insbesondere die Oxide von gelber, roter, brauner, schwarzer Farbe), Titandioxid, Zinkoxid, Ceroxid, Zirconiumoxid, Chromoxid; Manganviolett, Ultramarinblau, Preussisch Blau, Ultramarin und Eisenblau, Bismutoxidchlorid, Perlmutt, mit Titan oder Bismutoxidchlorid überzogene Glimmer-Pigmente, farbige Perlglanzpigmente, beispielsweise Titan-Glimmer-Pigmente mit Eisenoxiden, Titan-Glimmer-Pigmente, insbesondere mit Eisenblau oder Chromoxid, Titan-Glimmer-Pigmente mit einem organischen Pigment vom vorgenannten Typ, sowie Perlglanzpigmente auf der Basis von Bismutoxidchlorid, Ruß, die Pigmente vom Typ D & C, wie D&C Red N° 5, 6, 7, 10, 11, 12, 13, 34, D&C Yellow Lake N°5 und D&C Red Lake N° 2 und die Lacke auf der Basis von Cochenillerot, Barium, Strontium, Calcium und Aluminium und deren Gemische.

Zur Einstellung der rheologischen Eigenschaften können Verdickungsmittel als Additive eingesetzt werden. Erfindungsgemäß besonders vorteilhaft ist die Konzentration von Verdickungsmittel ≥ 0 und ≤ 20 Gew.-%, besonders vorteilhaft ≥ 0,5 und < 15 Gew.-%, ganz besonders vorteilhaft von ≥1 und ≤ 10 Gew.-%, jeweils bezogen auf das Gesamtgewicht der Zusammensetzung.

Vorteilhaft Verdickungsmittel sind organomodifizierte Tone wie organomodifizierte Bentonite, Stearalkonium Bentonite, organomodifizierte Hectorite, Stearalkonium Hectorite, oder organomodifizierte Montmorillonite, hydrophobe pyrogene Kieselsäure, wobei die Silanolgruppen mit Trimethylsiloxygruppen substituiert sind (AEROSIL® R812 der Firma Evonik) oder mit Dimethylsiloxygruppen oder Polydimethylsiloxan (AEROSIL® R972, AEROSIL® R974 von Evonik, CAB-O-SIL® TS-530, CAB-O-SIL® TS-610, "CAB-O-SIL® TS-720 von Cabot), Polysaccharide Alkylether (wie in EP 898958-A beschrieben).

Besonders vorteilhaft werden die organomodifizierte Tone ausgewählt aus der Gruppe von Quaternium-18 bentonite (Bentone® 3, Bentone® 38, Bentone® 38V der Firma Elementis, Tixogel VP Claytone 40, Claytone SO der Firma Southern Clay), Stearalkonium bentonite (Bentone®27V der Firma Elementis, Tixogel LG, Claytone AF der Firma Southern Clay), Quaternium-18/Benzalkonium bentonite (Claytone HT der Firma Southern Clay).

Die erfindungsgemäßen Zusammensetzungen können Füllstoffe enthalten. Unter Füllstoffe sind farblose oder weiße, mineralische oder synthetische, in dem physiologisch akzeptablen Medium der erfindungsgemäßen Zusammensetzung unlösliche, lamellare, sphärische oder längliche inerte Partikel zu verstehen, welche beispielsweise die rheologischen Eigenschaften und die Textur Zusammensetzung modifizieren.

Die Füllstoffe können in der erfindungsgemäßen Zusammensetzung in beispielsweise in einer Menge von ≥ 0 und ≤ 50 Gew.-% und vorzugsweise von ≥ 0,5 bis ≤ 30 Gew.-%, bezogen auf das Gesamtgewicht der Zusammensetzung, enthalten sein.

Vorteilhafte partikuläre Füllstoffe im Sinne der vorliegenden Erfindung sind Talkum, Glimmer (Mica), Siliciumdioxid, Kaolin, Stärke und deren Derivaten (beispielweise Tapiocastärke, Distärkephosphat, Aluminium- bzw. Natrium-Stärke Octenylsuccinat und dergleichen), pyrogene Kieselsäure, Pigmente, die weder hauptsächlich UV-Filter-noch färbende Wirkung haben (wie z.B. Bornitrid etc.), Bornitrid, Calciumcarbonat, Dicalciumphosphat, Magnesiumcarbonat, Magnesiumhydrogencarbonat, Hydroxyapatite, mikrokristalline Cellulose, Pulver von synthetischen Polymeren, wie Polyamide (beispielsweise die unter der Handelsbezeichnung "Nylon®" erhältlichen Polymeren), Polyethylen, Poly-β-alanin, Polytetrafluorethylen ("Teflon®"), Polyacrylat, Polyurethan, Lauroyl-lysine, Silikonharz (beispielsweise die unter der Handelsbezeichnung "Tospearl®" der Firma Momentive Performance Materials. erhältlichen Polymeren), Hohlpartikel von Polyvinyliden/Acrylonitrile (Expancel® der Firma Akzo Nobel) oder Hohlpartikel von Siliciumoxid (Silica Beads® der Firma MAPRECOS).

Vorteilhaft an der erfindungsgemäßen Zusammensetzung ist auch, dass Fingernägel pflegende Zusätze eingearbeitet werden können. Geeignet sind beispielsweise Vitamine B5, E und C und deren Derivativen sowie Dimethyloxobenzodioxasilane, Calciumchlorid, Calciumpantothenat, Panthenol, Proteine, Ceramide, Myrrhe, Pflanzenextrakte, Aminosäure Öle, wie zum Beispiel Cystein und deren Salzen und Derivate, Cystein, Glutathion, Biotin, Harnstoff und Dimethylharnstoff., alpha-Hydroxysäuren, wie Citronensäure und Ascorbinsäure, UV-Schutzmittel, wie Benzophenone-1, Benzophenone-3, Benzyl Salicylate, Etocrylene, Drometrizole, Butylmethoxydibenzoylmethane und härtende Zusätze wie Formaldehyd und Hydrolysate aus Chitin und/oder Keratin. Auch antimykotische Zusätze sind möglich.

In einer bevorzugten Ausführungsform der Erfindung enthält die Nagellackzusammensetzung einen Weichmacher A), einen Filmbildner B), gegebenenfalls wenigstens einen weiteren von A) unterschiedlichen Weichmacher C), gegebenenfalls wenigstens einen weiteren von B) verschiedenen Filmbildner D), ein physiologisch akzeptables Medium E) und gegebenenfalls Additive F).

In einer besonders bevorzugten Ausführungsform der Erfindung enthält die Nagellackzusammensetzung einen Weichmacher A), einen Filmbildner B), gegebenenfalls wenigstens einen weiteren von A) unterschiedlichen Weichmacher C), wenigstens einen weiteren von B) verschiedenen Filmbildner D), ein physiologisch akzeptables Medium E) und gegebenenfalls Additive F).

In einer ganz besonders bevorzugten Ausführungsform der Erfindung besteht die Nagellackzusammensetzung aus einem Weichmacher A), einem Filmbildner B), gegebenenfalls wenigstens einem weiteren von A) unterschiedlichen Weichmacher C), gegebenenfalls wenigstens einem weiteren von B) verschiedenen Filmbildner D), einem physiologisch akzeptablen Medium E) und gegebenenfalls Additiven F).

In einer weiterhin bevorzugten Ausführungsform enthält die Nagellackzusammensetzung > 0,1 und ≤ 20 Gew.-% des Weichmachers A), ≥ 5 und ≤ 30 Gew.-% des Filmbildners B), ≥ 0 und ≤ 25 Gew.-% des Weichmachers C), ≥ 0 und ≤ 30 Gew.-% des Filmbildners D), ≥ 10 und ≤ 95 Gew.-% des physiologisch akzeptablen Mediums E) und ≥ 0 und ≤ 20 Gew.-% an Additiven F), wobei sich die Angaben jeweils auf das Gesamtgewicht der Nagellackzusammensetzung beziehen und die Summe der Komponenten A) bis F) sich zu 100 Gew.-% ergänzt.

Vorteilhafterweise liegt der Anteil der Summe der Filmbildner B) und D) dabei bevorzugt bei ≥ 5 und ≤ 60 Gew.-% und der Anteil der Summe der Weichmacher A) und C) bevorzugt bei ≥ 0,1 und ≤ 30 Gew.-%, jeweils bezogen auf die gesamte Nagellackformulierung.

In einer weiterhin besonders bevorzugten Ausführungsform enthält die Nagellackzusammensetzung > 1 und ≤ 10 Gew.-% des Weichmachers A), > 10 und ≤ 20 Gew.-% des Filmbildners B), ≥ 1 und ≤ 5 Gew.-% des Weichmachers C), ≥ 1 und ≤ 15 Gew.-% des Filmbildners D), ≥ 20 und ≤ 70 Gew.-% des physiologisch akzeptablen Mediums E) und ≥ 0,5 und ≤ 10 Gew.-% an Additiven F), wobei sich die Angaben jeweils auf das Gesamtgewicht der Nagellackzusammensetzung beziehen und die Summe der Komponenten A) bis F) sich zu 100 Gew.-% ergänzt.

Vorteilhafterweise liegt der Anteil der Summe der Filmbildner B) und D) dabei bevorzugt bei ≥ 11 und ≤ 35 Gew.-% und der Anteil der Summe der Weichmacher A) und C) bevorzugt bei ≥ 3 und ≤ 12 Gew.-%, jeweils bezogen auf die gesamte Nagellackformulierung.

Bei den erfindungsgemäßen Nagellackzusammensetzungen kann es sich auch um durch UV-Strahlung aushärtbare Systeme handeln. Bevorzugt handelt es sich jedoch um nicht UVhärtbare Systeme.

Die erfindungsgemäße Nagellackzusammensetzung ist bevorzugt abgefüllt in Gefäßen mit einem Füllvolumen von ≤ 50 ml, bevorzugt ≤ 20 ml.

Ein weiterer Gegenstand der Erfindung ist eine Beschichtung erhältlich aus einer erfindungsgemäßen Nagellackzusammensetzung. Unter dieser Beschichtung ist insbesondere ein Film zu verstehen, der sich beim Auftragen der Nagellackzusammensetzung auf ein Substrat ausbildet und anschließend aushärtet. Dabei kann die Aushärtung des Films ohne Hilfsmittel, aber auch unter zu Hilfenahme beispielsweise einer UV-Lampe erfolgen.

Die aus der erfindungsgemäßen Nagellackformulierung erhältliche Beschichtung weist bevorzugt eine Pendelhärte von ≥ 20 König/s und ≤ 50 König/s, bevorzugt ≥ 30 König/s und ≤ 45 König/s auf. Die Pendelhärte wird dabei wie im Methodenteil der Beispiele angegeben bestimmt.

Des Weiteren weist die aus der erfindungsgemäßen Nagellackformulierung erhältliche Beschichtung bevorzugt einen Glanz ≥ 70 und ≤ 99, besonders bevorzugt von ≥ 90 und ≤ 98 auf. Die Bestimmung des Glanzes erfolgt dabei gemäß DIN 67530 mittels eines Glanzmesserätes "micro-haze plus" der Fa. BYK Gardner GmbH, Deutschland, nach Aufzug (200 µm nass) und Trocknung bei 23°C der jeweiligen Zusammensetzung nach 24 Stunden auf eine schwarz/weiß Leneta Karte. Der Glanz wird bei einem Winkel von 60° gemessen.

Weiterhin ist die aus der erfindungsgemäßen Nagellackformulierung erhältliche Beschichtung bevorzugt beständig gegen Wasser, das heißt insbesondere ist der erhaltene, ausgehärtete Beschichtungsfilm nach 4h in Wasser bei 40 °C noch vollständig, besonders bevorzugt ist der Film dann noch vollständig und auch klar. Die Wasserbeständigkeit wird dabei wie im Methodenteil der Beispiele angegeben bestimmt.

Ebenfalls Gegenstand der Erfindung ist ein Substrat beschichtet mit einer erfindungsgemäßen Beschichtung. Bei dem Substrat handelt es sich dabei bevorzugt um künstliche Nägel, die bereits am menschlichen Körper befestigt oder zur Befestigung am menschlichen Körper vorgesehen sind. Solche künstlichen Nägel basieren beispielsweise auf Materialien wie synthetischen Kunststoffen.

Ein weiterer Gegenstand der Erfindung ist ein kosmetisches Verfahren zum Beschichten von Finger- und/oder Fußnägeln, bei dem eine erfindungsgemäße Nagellackzusammensetzung auf Finger- und/oder Fußnägel aufgetragen wird. Unter Finger- und Fußnägeln sind dabei künstliche Nägel, die bereits am menschlichen Körper befestigt oder zur Befestigung am menschlichen Körper vorgesehen sind zu verstehen.

Die Nagellackzusammensetzung wird dabei mit einem Hilfsmittel auf die Finger- und/oder Fußnägel aufgetragen. Bei dem Hilfsmittel handelt es sich insbesondere um einen feinen Pinsel. Nach dem Auftragen bildet sich auf dem Nagel bevorzugt ein Film aus. Dieser Film härtet dann vorzugsweise aus. Bevorzugt härtet der Film dabei bei 23 °C innerhalb von ≤ 430 s, besonders bevorzugt innerhalb ≤ 410 s aus. Die Trocknungszeit wird dabei wie im Methodenteil der Beispiele angegeben bestimmt.

Die vorliegende Erfindung wird an Hand der folgenden Beispiele erläutert.

### Beispiele:

### Methoden:

Die Bestimmung der Pendelhärten erfolgte an einem Pendelhärtemessgerät "König-Pendulum-Hardness-Tester" der Fa. BYK Gardner GmbH, Deutschland nach DIN EN ISO 1522, nach Aufzug (240 µm nass) und Trocknung der jeweiligen Zusammensetzung während der Nacht (16 Stunden) bei 23°C und 50% relativer Luftfeuchte auf eine Glasplatte. Es wurde jeweils der Mittelwert aus 3 Messungen gebildet.

Die Festlegung der Trocknungszeit bei Raumtemperatur wurde nach Auftrag der Zusammensetzung auf eine Glasplatte (120 µm nass) mittels eines Trocknungszeitbestimmungsgerätes "drying time recorder" der Fa. BYK Gardner GmbH, Deutschland gemäß ASTM D5895 bestimmt. Die Trocknungszeit "to the touch" wurde ermittelt.

Zur Messung der Wasserbeständigkeit wurden Glasplatten nach Auftrag der Zusammensetzung (240 µm) und 24 Stunden Trocknung bei 23°C und 50% relativer Luftfeuchte in ein Wasserbad bei 40°C für einen Zeitraum von 4 Stunden eingetaucht. Die Wasserbeständigkeit wurde qualitativ bestimmt.

Die Anteile der Komponenten werden jeweils in Gew.-% angegeben und sind immer bezogen auf die Menge an Gesamtzusammensetzung.

### Verwendete Substanzen und Abkürzungen:

- Kollodium:: Mischung aus 50 Gew.-% Butylacetat, 26 Gew.-% Ethylacetat und 24 Gew.-% Nitrocellulose (die eingesetzte Nitrocellulose enthält 20 Gew.% Isopropanol).
- Desmophen® C1100:: Polyestercarbonatpolyol mit einer zahlenmittleren Molmasse von 1000 g/mol und einer OH-Funktionalität von 2; Handelsprodukt der Bayer MaterialScience AG (Abkürzung: D C1100)
- Desmophen® 3100 XP:: Polycarbonatpolyol mit einer zahlenmittleren Molmasse von 1000 g/mol und einer OH-Funktionalität von 2; Handelsprodukt der Bayer MaterialScience AG (Abkürzung: D 3100 XP)
- Desmophen® 680 BA:: Verzweigtes, Hydroxylgruppen-haltiges Polyesterpolyol mit einem Hydroxylgehalt von ca. 2,2% (nach DIN 53240/2) und einer Viskosität von ca. 3000 mPas (bei 23°C, nach DIN EN ISO 3219/A.3), gelöst in Butylacetat (die Kennzahlen beziehen sich auf die Lösung mit 70% nicht-flüchtigem Anteil nach DIN EN ISO 3251, gemessen bei 125°C) Handelsprodukt der Bayer MaterialScience AG (Abkürzung: D 680 BA)
- Desmophen® 670 BA:: leicht verzweigtes, Hydroxylgruppen-haltiges Polyesterpolyol mit einem Hydroxylgehalt von ca. 3,5% (nach DIN 53240/2) und einer Viskosität von ca. 2800 mPas (bei 23°C, nach DIN EN ISO 3219/A.3), gelöst in Butylacetat (die Kennzahlen beziehen sich auf die Lösung mit 80% nicht-flüchtigem Anteil nach DIN EN ISO 3251, gemessen bei 125°C), Handelsprodukt der Bayer Material-Science AG (Abkürzung: D 670 BA)
- Desmophen® 800 BA:: hochverzweigtes, Hydroxylgruppen-haltiges Polyesterpolyol mit einem Hydroxylgehalt von ca. 6,9% (nach DIN 53240/2) und einer Viskosität von ca. 3500 mPas (bei 23°C, nach DIN EN ISO 3219/A.3), gelöst in Butylacetat (die Kennzahlen beziehen sich auf die Lösung mit 80% nicht-flüchtigem Anteil nach DIN EN ISO 3251, gemessen bei 125°C), Handelsprodukt der Bayer Material-Science AG (Abkürzung: D 800 BA)

### Untersuchung der weichmachenden Wirkung:

Es wurden Nagellackformulierungen basierend auf Kollodium (Filmbildner 1), Butylacetat (physiologisch akzeptables Medium) und verschiedenen Weichmachern (ATC, Glycerincarbonat, Propylencarbonat und Desmophen® C1100) hinsichtlich ihrer Pendelhärte untersucht. Je geringer die Härte des aus der jeweiligen Zusammensetzung gebildeten Films ist, desto stärker ist die weichmachende Wirkung der eingesetzten Stoffe. Tabelle 1 gibt die Ergebnisse dieser Untersuchungen an. Es ist ersichtlich, dass Propylencarbonat als Weichmacher für Nagellackformulierungen ungeeignet ist und mit Polyesterpolyolen schlechtere Weichmacherwirkungen erzielt werden.

**Tabelle 1:**

| **V** | **Kollodium** | **ATC** | **Glycerincarbonat** | **Propylencarbonat** | **D C 1100** | **Butylacetat** | **D 680 BA** | **D 670 BA** | **D 800 BA** | **Pendelhärte [König/s]** |
|---|---|---|---|---|---|---|---|---|---|---|
| 1 | 50 | 10 | | | | 40 | | | | 7 |
| 2 | 50 | | 10 | | | 40 | | | | 7 |
| 3 | 50 | | | 10 | | 40 | | | | 208 |
| 4 | 50 | | | | 10 | 40 | | | | 7 |
| 5 | 50 | | | | | 40 | 10 | | | 88 |
| 6 | 50 | | | | | 40 | | 10 | | 224 |
| 7 | 50 | | | | | 40 | | | 10 | 73 |

### Untersuchung von Trocknungszeit und Wasserbeständigkeit:

Es wurden Nagellackformulierungen basierend auf Kollodium (Filmbildner 1), Desmophen® 680 BA (Filmbildner 2), Butylacetat (physiologisch akzeptables Medium) und verschiedenen Weichmachern (Glycerincarbonat, Desmophen® C1100 und Desmophen® 3100 XP) hinsichtlich ihrer Trocknungszeit und Wasserfestigkeit untersucht. Die Formulierungen wurden dabei jeweils so eingestellt, dass sie eine Pendelhärte zwischen 35 und 45 König/s aufweisen. Tabelle 2 fasst die Zusammensetzungen und die beobachteten Ergebnisse zusammen. Es hat sich überraschend gezeigt, dass die aus den erfindungsgemäßen Nagellackzusammensetzungen 6 und 7 gebildeten Filme sowohl eine etwas verbesserte Trocknungszeit gegenüber den Filmen des Vergleichsversuch 5, als auch eine deutlich bessere Wasserbeständigkeit aufweisen.

**Tabelle 2:**

| **V** | **Kollodium** | **D 680 BA** | **Glycerincarbonat** | **D C 1100** | **D 3100 XP** | **Butylacetat** | **Pendelhärte [König/s]** | **Trocknungszeit [s]** | **Wasserbeständigkeit** |
|---|---|---|---|---|---|---|---|---|---|
| 5* | 50 | 10 | 5 | | | 35 | 35 | 446 | zerstörter, milchiger Film |
| 6 | 50 | 10 | | 6 | | 34 | 44 | 381 | klarer, vollständiger Film |
| 7 | 50 | 10 | | | 7 | 33 | 41 | 406 | klarer, vollständiger Film |

| | | | | | | | | | |
|---|---|---|---|---|---|---|---|---|---|
| * Vergleichsversuch | | | | | | | | | |

## Patentansprüche

1. Nagellackzusammensetzung, enthaltend einen Weichmacher A) und einen Filmbildner B), **dadurch gekennzeichnet, dass** der Weichmacher A) mindestens zwei Carbonatstruktureinheiten der Formel (I), ein zahlenmittleres Molekulargewicht von ≥ 450 g/mol und ≤ 6000 g/mol, bestimmt durch Gelpermeationschromatographie gegenüber Polystyrol-Standards in Tetrahydrofuran bei 23°C gemäß DIN 55672-1, und eine Hydroxylfunktionalität von ≥ 1,5 und ≤ 6 aufweist, 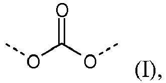 wobei der Weichmacher A) keine chemische Reaktion mit anderen Komponenten der Nagellackzusammensetzung eingeht.

2. Nagellackzusammensetzung gemäß Anspruch 1, **dadurch gekennzeichnet, dass** der Weichmacher A) keine Urethan und/oder Harnstoffgruppen aufweist.

3. Nagellackzusammensetzung gemäß einem der Ansprüche 1 oder 2, **dadurch gekennzeichnet, dass** diese wenigstens einen weiteren, von A) verschiedenen, Weichmacher C) enthält.

4. Nagellackzusammensetzung gemäß einem der Ansprüche 1 bis 3, **dadurch gekennzeichnet, dass** der Weichmacher A) eine Hydroxylfunktionalität von ≥ 1,9 und ≤ 2,1 aufweist.

5. Nagellackzusammensetzung gemäß einem der Ansprüche 1 bis 4, **dadurch gekennzeichnet, dass** der Weichmacher A) ausgewählt ist aus Polycarbonatpolyolen, Polyestercarbonatpolyolen und/oder Polyethercarbonatpolyolen.

6. Nagellackzusammensetzung gemäß einem der Ansprüche 1 bis 5, **dadurch gekennzeichnet, dass** der Anteil des Weichmacher A) ≥ 0,1 und ≤ 20 Gew.-% an der gesamten Nagellackformulierung beträgt.

7. Nagellackzusammensetzung gemäß einem der Ansprüche 1 bis 6, **dadurch gekennzeichnet, dass** der Filmbildner B) ausgewählt ist aus Nitrocellulosen, Celluloseacetobutyraten, Celluloseacetopropionaten, Celluloseethern und/oder Gemischen davon.

8. Nagellackzusammensetzung gemäß einem der Ansprüche 1 bis 7, **dadurch gekennzeichnet, dass** diese wenigstens einen weiteren, von B) verschiedenen, Filmbildner D) enthält.

9. Nagellackzusammensetzung gemäß Anspruch 8, **dadurch gekennzeichnet, dass** D) ein Polyesterpolyol ist.

10. Beschichtung erhältlich aus einer Nagellackzusammensetzung gemäß einem der Ansprüche 1 bis 9.

11. Künstliche Nägel beschichtet mit einer Beschichtung gemäß Anspruch 10.

12. Kosmetisches Verfahren zum Beschichten von künstlichen Finger- und/oder Fußnägeln, bei dem eine Nagellackzusammensetzung gemäß einem der Ansprüche 1 bis 9 auf künstliche Finger- und/oder Fußnägel aufgetragen wird.

13. Verwendung von Verbindungen, die mindestens zwei Carbonatstruktureinheiten der Formel (I), 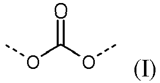 ein zahlenmittleres Molekulargewicht von ≥ 450 g/mol und ≤ 6000 g/mol, bestimmt durch Gelpermeationschromatographie (GPC) gegenüber Polystyrol-Standards in Tetrahydrofuran bei 23°C gemäß DIN 55672-1, und eine Hydroxylfunktionalität von ≥ 1,5 und ≤ 6 aufweisen, als Weichmacher in Nagellackzusammensetzungen, **dadurch gekennzeichnet, dass** der Weichmacher keine chemische Reaktion mit anderen Komponenten der Nagellackzusammensetzung eingeht.

## Claims

1. Nail varnish composition comprising a plasticizer A) and a film former B), **characterized in that** the plasticizer A) has at least two carbonate structural units of the formula (I), a number-average molecular weight of ≥ 450 g/mol and ≤ 6000 g/mol, determined by gel permeation chromatography against polystyrene standards in tetrahydrofuran at 23°C in accordance with DIN 55672-1, and a hydroxyl functionality of ≥ 1.5 and ≤ 6, 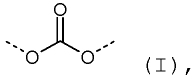 wherein the plasticizer A) does not enter into a chemical reaction with other components of the nail varnish composition.

2. Nail varnish composition according to Claim 1, **characterized in that** the plasticizer A) has no urethane and/or urea groups.

3. Nail varnish composition according to one of Claims 1 and 2, **characterized in that** it comprises at least one further plasticizer C) different from A).

4. Nail varnish composition according to one of Claims 1 to 3, **characterized in that** the plasticizer A) has a hydroxyl functionality of ≥ 1.9 and ≤ 2.1.

5. Nail varnish composition according to one of Claims 1 to 4, **characterized in that** the plasticizer A) is selected from polycarbonate polyols, polyester carbonate polyols and/or polyethercarbonate polyols.

6. Nail varnish composition according to one of Claims 1 to 5, **characterized in that** the fraction of the plasticizer A) is ≥ 0.1 and ≤ 20% by weight of the total nail varnish formulation.

7. Nail varnish composition according to one of Claims 1 to 6, **characterized in that** the film former B) is selected from nitrocelluloses, cellulose acetobutyrates, cellulose acetopropionates, cellulose ethers and/or mixtures thereof.

8. Nail varnish composition according to one of Claims 1 to 7, **characterized in that** this comprises at least one further film former D) different from B).

9. Nail varnish composition according to Claim 8, **characterized in that** D) is a polyesterpolyol.

10. Coating obtainable from a nail varnish composition according to one of Claims 1 to 9.

11. Artificial nails coated with a coating according to Claim 10.

12. Cosmetic method for coating artificial fingernails and/or toenails, in which a nail varnish composition according to one of Claims 1 to 9 is applied to artificial fingernails and/or toenails.

13. Use of compounds which have at least two carbonate structural units of the formula (I), 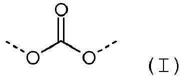 a number-average molecular weight of ≥ 450 g/mol and ≤ 6000 g/mol, determined by gel permeation chromatography (GPC) against polystyrene standards in tetrahydrofuran at 23°C in accordance with DIN 55672-1, and a hydroxyl functionality of ≥ 1.5 and ≤ 6, as plasticizers in nail varnish compositions, **characterized in that** the plasticizer does not enter into a chemical reaction with other components of the nail varnish composition.

## Revendications

1. Composition de vernis à ongle, contenant un plastifiant A) et un agent filmogène B), **caractérisée en ce que** le plastifiant A) comprend au moins deux unités structurales carbonate de formule (I), présente un poids moléculaire moyen en nombre ≥ 450 g/mol et ≤ 6 000 g/mol, déterminé par chromatographie par perméation de gel par rapport à des étalons de polystyrène dans du tétrahydrofurane à 23 °C selon DIN 55672-1, et une fonctionnalité hydroxyle ≥ 1,5 et ≤ 6, 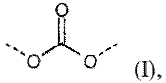 le plastifiant A) ne réalisant pas de réaction chimique avec d'autres composants de la composition de vernis à ongles.

2. Composition de vernis à ongles selon la revendication 1, **caractérisée en ce que** le plastifiant A) ne comprend pas de groupes uréthane et/ou urée.

3. Composition de vernis à ongles selon l'une quelconque des revendications 1 ou 2, **caractérisée en ce que** celle-ci contient au moins un autre plastifiant C) différent de A).

4. Composition de vernis à ongles selon l'une quelconque des revendications 1 à 3, **caractérisée en ce que** le plastifiant A) présente une fonctionnalité hydroxyle ≥ 1,9 et ≤ 2,1.

5. Composition de vernis à ongles selon l'une quelconque des revendications 1 à 4, **caractérisée en ce que** le plastifiant A) est choisi parmi les polycarbonate-polyols, les polyester-carbonate-polyols et/ou les polyéther-carbonate-polyols.

6. Composition de vernis à ongles selon l'une quelconque des revendications 1 à 5, **caractérisée en ce que** la proportion du plastifiant A) est ≥ 0,1 et ≤ 20 % en poids de la formulation de vernis à ongles totale.

7. Composition de vernis à ongles selon l'une quelconque des revendications 1 à 6, **caractérisée en ce que** l'agent filmogène B) est choisi parmi les nitrocelluloses, les acétobutyrates de cellulose, les acétopropionates de cellulose, les éthers de cellulose et/ou leurs mélanges.

8. Composition de vernis à ongles selon l'une quelconque des revendications 1 à 7, **caractérisée en ce que** celle-ci contient au moins un autre agent filmogène D) différent de B).

9. Composition de vernis à ongles selon la revendication 8, **caractérisée en ce que** D) est un polyester-polyol.

10. Revêtement pouvant être obtenu à partir d'une composition de vernis à ongles selon l'une quelconque des revendications 1 à 9.

11. Ongles artificiels, revêtus avec un revêtement selon la revendication 10.

12. Procédé cosmétique pour revêtir des ongles artificiels des mains et/ou des pieds, selon lequel une composition de vernis à ongles selon l'une quelconque des revendications 1 à 9 est appliquée sur des ongles artificiels des mains et/ou des pieds.

13. Utilisation de composés contenant au moins deux unités structurales carbonate de formule (I) 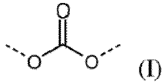 présentant un poids moléculaire moyen en nombre ≥ 450 g/mol et ≤ 6 000 g/mol, déterminé par chromatographie par perméation de gel (CPG) par rapport à des étalons de polystyrène dans du tétrahydrofurane à 23 °C selon DIN 55672-1, et une fonctionnalité hydroxyle ≥ 1,5 et ≤ 6, en tant que plastifiant dans des compositions de vernis à ongles, **caractérisée en ce que** le plastifiant ne réalise pas de réaction chimique avec d'autres composants de la composition de vernis à ongles.
